# EUROPEAN PATENT APPLICATION

(11) **EP 4 177 669 A1**
(43) Date of publication of application: **10.05.2023**
(21) Application number: 21866665.9
(22) Date of filing: 03.09.2021
(51) Int. Cl.: G02C 7/04, C08J 7/04, C08J 7/12

(54) **COATED SILICONE MEMBER MANUFACTURING METHOD**

(30) Priority: 14.09.2020 JP 2020153532
(71) Applicant: Toray Industries, Inc., Tokyo 103-8666 (JP)
(72) Inventor: IIMORI, Hirokazu, Okazaki-shi, Aichi 444-8522 (JP); NAKAMURA, Masataka, Otsu-shi, Shiga 520-8558 (JP)
(74) Representative: Prüfer & Partner mbB Patentanwälte · Rechtsanwälte
(86) International application number: PCT/JP2021/032408
(87) International publication number: WO 2022/054706

(57) **Abstract**

The purpose of the present invention is to provide a coated silicone member manufacturing method which enables, even when a highly water-repellent silicone member having a water surface contact angle of more than 110° is processed at a low temperature, formation of, on the surface, a coating having a sufficiently low surface contact angle with respect to an aqueous solution containing a salt and a protein. The present invention provides a coated silicone member manufacturing method comprising: (A) a degradative reaction step for degrading the surface of a silicone member; (B) a contact step b for bringing a solution b containing a polycarbodiimide into contact with the surface-degraded silicone member; and (C) a contact step c for bringing a solution c containing a polymer C into contact with the surface-degraded silicone member. The polymer C has an alkyl amide group and hydroxyl group.

## Description

### TECHNICAL FIELD

The present invention relates to a method for producing a coated silicone member.

### BACKGROUND ART

The silicone member is inexpensive, has excellent heat resistance, cold resistance, and insulation resistance, and has rubber elasticity and flexibility in a wide temperature range. In addition, since it is a material that is resistant to ultraviolet rays and heat and is capable of heat sterilization treatment, light sterilization treatment, gamma ray sterilization, and EOG sterilization, it is also used as a member of a medical device such as a medical tube, a catheter component, a valve of a medical device, a check valve, a hemostatic valve, a contact lens, a cell culture sheet, a scaffold material for tissue regeneration, and a medical electrode for skin.

On the other hand, the silicone member has high water repellency. When the silicone member is mainly used as a member of a medical device, the silicone member is brought into contact with water, blood, physiological saline, sweat, tear, or the like, so that high water repellency of the silicone member may be a problem. In the case of being brought into contact with a mucous membrane in a living body or in the case of being worn on the eye, in order to improve biocompatibility, it is important to improve the affinity of the surface for water, blood, physiological saline, sweat, and tear, that is, to form a coating on the surface of the silicone member.

As a method for forming a hydrophilic coating on the surface of various silicone members, a method for crosslinking a polymer having a carboxyl group and a material having an epoxide group (Patent Document 1), a method by heat treatment using a polymer having an amide group (Patent Document 2), a method for forming a coating using a copolymer of N-vinylpyrrolidone/acrylate salt and a crosslinking agent (Patent Document 3), and the like are known.

### PRIOR ART DOCUMENTS

### PATENT DOCUMENTS

Patent Document 1: Published Japanese Translation No. 2014-533381 of the PCT International Publication
Patent Document 2: International Publication No. 2019/031477
Patent Document 3: Published Japanese Translation No. 2019-501270 of the PCT International Publication

### SUMMARY OF THE INVENTION

### PROBLEMS TO BE SOLVED BY THE INVENTION

However, in the known method, when treatment is attempted at a relatively low temperature such as 80°C or lower on a silicone member having very high water repellency in which the surface contact angle with respect to water exceeds 110°, a coating having a sufficiently low surface contact angle with respect to an aqueous solution containing a salt and a protein, such as those contained in blood or tear, may not be formed.

Therefore, an object of the present invention is to provide a method for producing a coated silicone member in which a coating showing a sufficiently low surface contact angle with respect to an aqueous solution containing a salt and a protein is formed on a surface thereof even when a highly water-repellent silicone member having a surface contact angle with water of more than 110° is treated at a lower temperature.

### SOLUTIONS TO THE PROBLEMS

In order to solve the above problems, a method for producing a coated silicone member of the present invention includes: (A) a decomposition reaction step of decomposing a surface of a silicone member; (B) a contact step b of bringing the silicone member whose surface is decomposed into contact with a solution b containing polycarbodiimide; and (C) a contact step c of bringing the silicone member whose surface is decomposed into contact with a solution c containing a polymer C, wherein the polymer C has an alkylamide group and a hydroxyl group.

### EFFECTS OF THE INVENTION

According to the method for producing a coated silicone member of the present invention, even when a highly water-repellent silicone member having a surface contact angle with respect to water of more than 110° is treated at a relatively low temperature such as 80°C or less, a coating exhibiting a sufficiently low surface contact angle with respect to an aqueous solution containing a salt and a protein can be formed on the surface of the coated silicone member.

### EMBODIMENTS OF THE INVENTION

### <Silicone member>

The silicone member subjected to the decomposition reaction step included in the method for producing a coated silicone member of the present invention refers to a member made of a silicone resin having a repeating unit of - Si(Rᵢ)²-O- (where R₁ is an optional organic group). Examples of the silicone resin include silicone rubber, silicone resin, acrylic silicone, silicone hydrogel, and the like.

When the coated silicone member is used for a medical tube, a contact lens, a cell culture sheet, a scaffold material for tissue regeneration, a medical electrode for skin, or the like, silicone hydrogel is preferable among silicone resins from the viewpoint of having excellent flexibility and high wettability. For example, when the silicone hydrogel is used as the silicone member for ophthalmic lens, its flexibility gives excellent comfort and high oxygen permeability enables wearing for a longer time, which is preferable.

### <(A) Decomposition reaction step>

The production method of the present invention includes a decomposition reaction step of decomposing the surface of the silicone member. Here, "decomposition" refers to chemical decomposition. Examples of the chemical decomposition of the surface of the silicone member include decomposition of a chemically crosslinked chain, decomposition of a silicone chain (a molecular chain composed of repeated bonds represented by -Si-O-), decomposition of a molecular chain other than a silicone chain, and decomposition of a functional group. Examples of the decomposition reaction step include a hydrolysis reaction step using an aqueous alkali solution composed of an alkali and water, a decomposition reaction step using atmospheric pressure plasma treatment, and a decomposition reaction step using vacuum plasma treatment.

A hydrolysis step using an aqueous alkali solution is preferable in that the treatment can be performed at a lower temperature using a simple apparatus. Here, the alkali refers to a compound exhibiting basicity in water. Examples of the alkali include organic alkaline compounds typified by tertiary amines or phosphines, inorganic alkaline compounds typified by potassium hydroxide, sodium hydroxide, calcium hydroxide, calcium carbonate, potassium hydrogen carbonate, sodium carbonate, sodium hydrogen carbonate, lithium hydrogen carbonate, or sodium dithionite (sodium hydrosulfite), and organic metal salt-based alkaline compounds such as sodium methoxide or t-butoxypotassium. Among them, an inorganic alkaline compound is preferable, potassium hydroxide, sodium hydroxide, calcium hydroxide, calcium carbonate, potassium hydrogen carbonate or sodium hydrogen carbonate is more preferable, potassium hydroxide, sodium hydroxide, calcium hydroxide or calcium carbonate is still more preferable, and sodium hydroxide is particularly preferable from the viewpoint of being inexpensive and having relatively high safety.

In the hydrolysis step using the aqueous alkali solution, a solvent other than water may be used in addition to the aqueous alkali solution. Examples of the solvent other than water include alcohols such as methanol, ethanol, isopropanol, n-propanol, n-butanol, ethylene glycol, diethylene glycol, and triethylene glycol, and isopropanol is preferable from the viewpoint of cost and safety. Examples of other solvents include hexane, cyclohexanone, acetone, dimethyl sulfoxide, and the like, and dimethyl sulfoxide is preferable because it has a high flash point, is easy to handle, and is easily mixed with an aqueous alkali solution.

The pH of the alkali solution used in the decomposition reaction step is preferably within the range of 8.0 to 14.0 because when the pH is too high, the effect of strength reduction due to the decomposition reaction inside the silicone member is large, and when the pH is too low, the surface cannot be sufficiently hydrolyzed. The pH is more preferably 8.5 or more, still more preferably 9.0 or more, even more preferably 9.2 or more, and particularly preferably 9.8 or more. In addition, pH is more preferably 13.9 or less, still more preferably 13.7 or less, even more preferably 13.5 or less, and particularly preferably 13.3 or less.

The pH of the alkaline solution can be measured using a pH meter (for example, Eutech pH 2700 (manufactured by Nikko Hansen & Co., Ltd.)). Specifically, the pH of the alkali solution can be determined by adding alkali to water and/or the solvent, then stirring the mixture at room temperature (20 to 25°C) for 30 minutes using a rotor, making the solution uniform, then measuring the pH, and rounding the measured value to the first decimal place.

The temperature in the case of heating the alkaline solution is preferably 50°C or higher, more preferably 70°C or higher, and still more preferably 90°C or lower, because if the temperature is too high, the influence of strength reduction due to the decomposition reaction inside the silicone member is large, and if the temperature is too low, the surface cannot be sufficiently hydrolyzed.

Examples of the plasma used in the decomposition reaction step using atmospheric pressure plasma treatment or the decomposition reaction step using vacuum plasma treatment include oxygen plasma, nitrogen plasma, argon plasma, hydrogen plasma, microwave plasma, and high frequency plasma. Argon plasma is preferable in that surfaces of many silicone members can be uniformly decomposed in a short time. Furthermore, vacuum plasma treatment using high frequency plasma is more preferable in that it is possible to generate a silanol group which is likely to hydrophilically interact with the polymer C or polycarbodiimide described later on the surface in a short time. The atmospheric pressure or the temperature in the case of performing the vacuum plasma treatment is preferably 200°C or lower, more preferably 150°C or lower, and still more preferably 100°C or lower in that deterioration of the silicone member does not occur. In order to uniformly decompose the surface of the silicone member, it is preferable that argon and oxygen are mixed in advance and introduced into a plasma treatment apparatus.

### <(B) Contact step b>

The production method of the present invention includes (B) a contact step b of bringing the silicone member into contact with a solution b containing polycarbodiimide. Here, the "polycarbodiimide" refers to a polymer compound having a carbodiimide group (-N=C=N-) structure in the molecule. The carbodiimide group preferably forms a part of the main chain of the polycarbodiimide in that the carbodiimide group can be easily synthesized. In addition, the polycarbodiimide preferably has a plurality of carbodiimide groups in one molecule from the viewpoint that a coated silicone member having a highly durable coating can be obtained. Examples of the repeating unit contained in the polycarbodiimide are shown in the following structural formulas (1) to (4). The terminal of the repeating unit contained in the polycarbodiimide as defined by the following structural formulas (1) to (4) may be sealed with another compound or a water-soluble resin. In the formulas (1) to (4), n is an integer representing the number of repetitions, and a symbol indicating that these formulas are repeating units. In Formula (4), m represents an integer of 1 to 20.

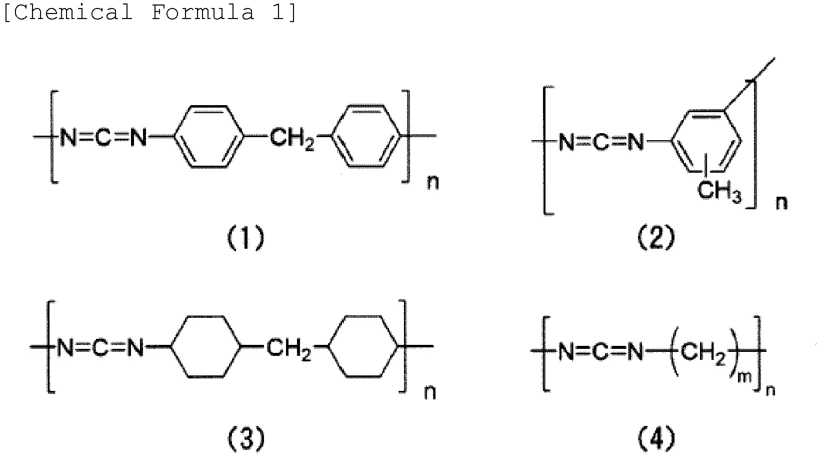

m in Formula (4) is preferably 3 or more, more preferably 4 or more, and still more preferably 5 or more from the viewpoint of availability or ease of synthesis of the raw material compound. In addition, m is preferably 18 or less, more preferably 16 or less, still more preferably 12 or less, and particularly preferably 6 or less.

The "weight average molecular weight" in the present specification refers to a weight average molecular weight in terms of polyethylene glycol or polyethylene oxide. The weight average molecular weight of the polycarbodiimide is preferably 10,000 or more, more preferably 30,000 or more, and still more preferably 45,000 or more. When the weight average molecular weight is large to a certain extent, the polycarbodiimide is sufficiently held on the surface than the inside of the silicone member, and a coated silicone member having a lower surface contact angle is obtained. In addition, a polycarbodiimide having a weight average molecular weight small to a certain extent is preferable from the viewpoint of low toxicity since the polycarbodiimide has high reactivity. The weight average molecular weight of the polycarbodiimide is preferably 1,000,000 or less, more preferably 200,000 or less, and still more preferably 100,000 or less. In addition, when the weight average molecular weight is small to a certain extent, the polycarbodiimide can be uniformly treated on the silicone member surface, and a uniform surface can be obtained without causing liquid dripping or a non-uniform liquid pattern. The weight average molecular weight can be measured by gel permeation chromatography (GPC).

In addition, when the concentration of the solution b containing polycarbodiimide is too high, the surface of the polymer C and the decomposed silicone member to be described later is unevenly coated, and the surface tends to have dripping and an uneven liquid pattern. On the other hand, when the concentration is too low, the surface of the silicone member cannot be sufficiently covered.

Therefore, the concentration of the polycarbodiimide in the solution b is preferably in the range of 0.001 to 5.000 mass%. The concentration of the polycarbodiimide is more preferably 0.010 mass% or more, and still more preferably 0.040 mass% or more. In addition, the concentration of the polycarbodiimide is more preferably 3.000 mass% or less, further preferably 2.000 mass% or less, further preferably 1.000 mass% or less, further preferably 0.700 mass% or less, further preferably 0.500 mass% or less, and particularly preferably 0.300 mass% or less.

The pH of the solution b is preferably weakly alkaline in that a highly durable coating can be formed. The pH of the solution b is preferably 7.0 to 10.0, more preferably 7.5 to 9.0, and still more preferably 7.8 to 8.5.

The temperature of the solution b is preferably 50°C or higher, and more preferably 60°C or higher in order to form a highly durable coating. On the other hand, in order to obtain a coated silicone member exhibiting a sufficiently low surface contact angle with respect to an aqueous solution containing a salt and a protein, the temperature is preferably 80°C or lower, more preferably 70°C or lower, and still more preferably 60°C or lower. In particular, it is particularly preferable to heat the solution to 50°C to 80°C after immersing the silicone member in the solution b at room temperature from the viewpoint that the silicone member does not react immediately after being brought into contact with the solution b, but gradually reacts with the polymer C described later or the surface of the silicone member whose surface is decomposed to form a uniform coating.

The solution b is preferably an emulsion from the viewpoint that the silicone member does not react immediately after being brought into contact with the solution b, but gradually reacts with the polymer C described later or the surface of the silicone member whose surface is decomposed to form a uniform coating.

Suitable examples of the solvent of the solution b include water, alcohols, ketones, ethers, aprotic polar solvents, and mixed solvents thereof. Among them, water and a mixed solvent of water and alcohols are preferable, and water is most preferable.

### <(C) Contact step c>

The production method of the present invention includes (C) a contact step c of contacting the silicone member with a solution c containing a polymer C, in which the polymer C is required to have an alkylamide group and a hydroxyl group.

Here, the alkylamide group refers to a functional group represented by the following General Formula (5).

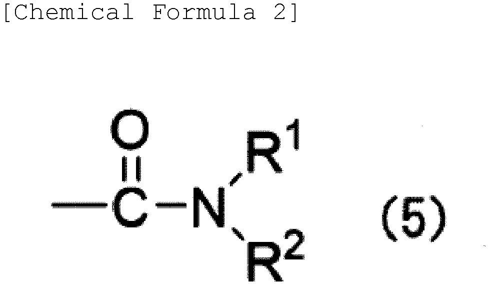

In the above General Formula (5), R¹ and R² each independently represent hydrogen or an alkyl group, but either R¹ or R² needs to be an alkyl group having 1 to 12 carbon atoms. It is preferable that R¹ and R² are both alkyl groups from the viewpoint of enhancing the long-term storage stability of the coated silicone member. As the alkyl group, a methyl group, an ethyl group, a propyl group, or an isopropyl group having 1 to 3 carbon atoms is preferable, and a methyl group is more preferable in that the surface contact angle of the coated silicone member can be lowered.

Examples of the polymer C having an alkylamide group and a hydroxyl group include a copolymer containing a monomer having an alkylamide group and a monomer having a hydroxyl group as monomer components.

As the monomer having an alkylamide group, N-methyl-N-vinylacetamide, N,N-dimethylacrylamide, N,N-diethylacrylamide, N-isopropylacrylamide, or a monomer having a (meth)acrylic group is preferable, and N,N-dimethylacrylamide is more preferable from the viewpoint of enhancing the surface lubricity of the obtained coated silicone member.

Examples of the monomer having a hydroxyl group include monomers having a substituent selected from the group consisting of a carboxylic acid group, a sulfonic acid group, an alcohol group, and a phenol group, and monomers having a carboxylic acid group or an alcohol group are preferable. Here, examples of the monomer having a carboxylic acid group include methacrylic acid, acrylic acid, and itaconic acid. Examples of the monomer having a hydroxyl group include 2-hydroxyethyl (meth)acrylate, 2-hydroxypropyl (meth)acrylate, and glycerol (meth)acrylate. Among them, a monomer having a carboxylic acid group, which has high hydrophilicity and capable of adjusting the hydrophilicity with a small amount is preferable.

Examples of the polymer C include a (meth)acrylic acid/N,N-dimethylacrylamide copolymer and a 2-acrylamido-2-methylpropanesulfonic acid/N,N-dimethylacrylamide copolymer, and a (meth)acrylic acid/N,N-dimethylacrylamide copolymer is preferable. A terpolymer or a multicomponent copolymer obtained by further adding different monomer components to these copolymers may be used as the polymer C. In that case, as the monomer component to be added, for example, hydrophilic (meth)acrylates such as 2-methoxyethyl (meth)acrylate, 2-ethoxyethyl (meth)acrylate, or 2-isopropoxyethyl (meth)acrylate, N-vinylpyrrolidone, N-vinylcaprolactam, N-vinylacetamide, N-methyl-N-vinylacetamide, N-vinylformamide, acryloylmorpholine, acrylamide, or the like is preferable.

The polymer C preferably has a 2-alkoxyethyl group from the viewpoint that the surface wettability of the coated silicone member obtained by the production method of the present invention can be maintained for a long time. The polymer C is preferably a terpolymer or a multipolymer obtained by copolymerizing (meth)acrylates having a 2-alkoxyethyl group. Examples of the (meth)acrylates having a 2-alkoxyethyl group include 2-methoxyethyl (meth)acrylate, diethylene glycol monomethyl ether (meth)acrylate, triethylene glycol monomethyl ether (meth)acrylate, and polyethylene glycol monomethyl ether (meth)acrylate, and monomers obtained by substituting a methoxy group of these monomers with an ethoxy group or an isopropoxy group. Examples of the 2-alkoxyethyl group include a 2-methoxyethyl group, a 2-ethoxyethyl group, and a 2-isopropoxyethyl group, and a 2-methoxyethyl group is more preferable.

The pH of the solution c is preferably acidic, more preferably 5.0 or less, still more preferably 3.5 or less from the viewpoint of having high durability and being capable of forming a coating having a low surface contact angle.

Suitable examples of the solvent of the solution c include water, alcohols, ketones, ethers, aprotic polar solvents, and mixed solvents thereof. Among them, water and a mixed solvent of water and alcohols are preferable, and water is most preferable.

The concentration of the polymer C in the solution c is preferably in the range of 0.001 to 5.000 mass%. The concentration of the polymer C is more preferably 0.010 mass% or more, and still more preferably 0.040 mass% or more. In addition, the concentration of the polymer C is more preferably 3.000 mass% or less, further preferably 2.000 mass% or less, further preferably 1.000 mass% or less, further preferably 0.700 mass% or less, further preferably 0.500 mass% or less, and particularly preferably 0.300 mass% or less.

The temperature of the solution c is preferably 50°C or higher, and more preferably 60°C or higher in order to form a highly durable coating. On the other hand, in order to obtain a coated silicone member exhibiting a sufficiently low surface contact angle with respect to an aqueous solution containing a salt and a protein, the temperature is preferably 100°C or lower, preferably 80°C or lower, more preferably 70°C or lower, and still more preferably 60°C or lower.

In the production method of the present invention, the decomposition reaction step (A), the contact step b (B), and the contact step c (C) are performed in the order of (A), (B), and (C), or in the order of (A), (C), and (B). It is more preferable to perform (A), (B), and (C) in this order. By performing these in this order, a coating having high durability is easily formed.

### <(D) Contact step d>

In the production method of the present invention according to another aspect, instead of the contact step b and the contact step c, (D) a contact step d of bringing the silicone member into contact with a solution d containing polycarbodiimide and a polymer C may be included. When the reaction between the polymer C and the carbodiimide is allowed to proceed not at room temperature but by heating, the pH of the prepared solution d is preferably alkaline, more preferably 7.0 to 13.5, still more preferably 8.0 to 12.0, and particularly preferably 9.5 to 10.5. The temperature of the solution d is preferably 50°C or higher, and more preferably 60°C or higher in order to form a highly durable coating. On the other hand, in order to obtain a coated silicone member exhibiting a sufficiently low surface contact angle with respect to an aqueous solution containing a salt and a protein, the temperature is preferably 80°C or lower, more preferably 70°C or lower, and still more preferably 60°C or lower. In particular, it is preferable to heat the solution to 50°C to 80°C after immersing the silicone member whose surface is decomposed in the solution d at room temperature from the viewpoint that the silicone member does not react immediately after being brought into contact with the solution d, but gradually reacts with the surface of the silicone member whose surface is decomposed to form a uniform coating.

In addition, the solution d is preferably an emulsion from the viewpoint that the silicone member whose surface is decomposed does not react immediately after being brought into contact with the solution d, but gradually reacts with the surface of the silicone member after the decomposition reaction step, so that a uniform and durable coating can be formed.

Suitable examples of the solvent of the solution d include water, alcohols, ketones, ethers, aprotic polar solvents, and mixed solvents thereof. Among them, water and a mixed solvent of water and alcohols are preferable, and water is most preferable.

The concentration of the polycarbodiimide in the solution d is preferably in the range of 0.001 to 5.000 mass%. The concentration of the polycarbodiimide is more preferably 0.010 mass% or more, and still more preferably 0.040 mass% or more. In addition, the concentration of the polycarbodiimide is more preferably 3.000 mass% or less, further preferably 2.000 mass% or less, further preferably 1.000 mass% or less, further preferably 0.700 mass% or less, further preferably 0.500 mass% or less, and particularly preferably 0.300 mass% or less.

The concentration of the polymer C in the solution d is preferably in the range of 0.001 to 5.000 mass%. The concentration of the polymer C is more preferably 0.010 mass% or more, and still more preferably 0.040 mass% or more. In addition, the concentration of the polymer C is more preferably 3.000 mass% or less, further preferably 2.000 mass% or less, further preferably 1.000 mass% or less, further preferably 0.700 mass% or less, further preferably 0.500 mass% or less, and particularly preferably 0.300 mass% or less.

The temperature of the solution d is preferably 50°C or higher, and more preferably 60°C or higher in order to form a highly durable coating. On the other hand, in order to obtain a coated silicone member exhibiting a sufficiently low surface contact angle with respect to an aqueous solution containing a salt and a protein, the temperature is preferably 100°C or lower, preferably 80°C or lower, more preferably 70°C or lower, and still more preferably 60°C or lower.

In the production method of the present invention according to the above aspect, the decomposition reaction step (A) and the contact step d (D) are performed in the order of (A) and (D). By performing in the order of (A) and (D), a coating having high durability is easily formed.

### <Polymer having carboxylic acid group>

The solution c and the solution d preferably further contain a polymer having a carboxylic acid group. By forming a composite of a polymer having a carboxylic acid group and a copolymer having an alkylamide structure, a durable coating can be formed. The polymer having a carboxylic acid group may be a homopolymer or a copolymer, but in the case of a copolymer, a polymer not containing an alkylamide structural unit is referred to as a "polymer having a carboxylic acid group". In terms of being capable of having a carboxylic acid group at high density, the polymer having a carboxylic acid group is preferably composed only of a monomer component having a carboxylic acid group, and more preferably a homopolymer.

Examples of the polymer having a carboxylic acid group include polymethacrylic acid, polyacrylic acid, polyvinylbenzoic acid, poly(thiophene-3-acetic acid), polymaleic acid, poly(meth)acryloyloxyethyl-succinic acid, poly(meth)acryloyloxyethyl hexahydrophthalic acid, poly(meth)acryloyloxyethyl-phthalic acid, poly(meth)acryloyloxyethyl-2-hydroxyethyl-phthalic acid, polynorbornenecarboxylic acid, and poly(meth)acryloyloxyethyl succinate, and salts these polymers.

The weight average molecular weight of the polymer having a carboxylic acid group contained in the solution c and the solution d is preferably 10,000 or more and 500,000 or less. The weight average molecular weight of the polymer having a carboxylic acid group is preferably 10,000 or more, more preferably 100,000 or more, and still more preferably 200,000 or more, in that a coating having high durability of surface lubricity against scrubbing. In addition, in order to form a uniform coating, it is preferably 500,000 or less, more preferably 400,000 or less, and still more preferably 300,000 or less. Any lower limit value and any upper limit value may be used in combination.

### <Fixing step>

The production method of the present invention may further include a fixing step of fixing the silicone member to the surface of the substrate (X). Here, the substrate is a molded body different from the silicone member, and may be a material such as various synthetic resins, natural resins, glass, ceramic, metal, or wood. As a method for fixing the silicone member to the surface of the substrate (X), for example, a method for molding the silicone member using a pair of molding molds (molds) and then removing only one mold is preferable. In this case, the molding and fixing step of the silicone member can be performed simultaneously. In this case, the other mold in which the silicone member remains fixed to the surface corresponds to the substrate (X). Furthermore, in this case, the contact step b, the contact step c, or the decomposition step may be performed while the silicone member is fixed to the surface of the substrate (X).

### <Application of coated silicone member>

The coated silicone member obtained by the production method of the present invention is suitably used as, for example, an intraocular lens, an artificial cornea, an ophthalmic lens such as a corneal inlay or a corneal onlay, a covering material for skin, a wound covering material, a protective material for skin, a drug carrier for skin, an infusion tube, a gas transport tube, a drainage tube, a blood circuit, a covering tube, a catheter, a stent, a sheath, a tube connector, or an access port.

### EXAMPLES

Hereinafter, the present invention will be specifically described with reference to Examples and the like, but the present invention is not limited thereto.

In Examples, the following was used.
MEA: 2-Methoxyethyl acrylate, manufactured by Tokyo Chemical Industry Co., Ltd.
DMAA: N,N-dimethylacrylamide, manufactured by Tokyo Chemical Industry Co., Ltd.
164B: Difunctional silicone monomer, X-22-164B (α,ω-bis(3-methacryloxy-propyl)-polydimethylsiloxane), functional group equivalent: 1,600, manufactured by Shin-Etsu Chemical Co., Ltd.
FM0711: Linear polymerizable silicone compound "Silaplane" (registered trademark) (polydimethylsiloxane monomethacrylate), average molecular weight: 1000, manufactured by JNC Corporation,
NB: Ultraviolet absorber 2-(2'-hydroxy-5'-methacryloyloxyethylphenyl)-2H-benzotriazole, manufactured by Tokyo Chemical Industry Co., Ltd.
RB: Colorant Reactive Blue 246 (1,4-bis[4-(2-methacryloxyethyl)phenylamino]anthraquinone), manufactured by Arran Chemical Co., LTD.
IC-819: Photoinitiator "IRGACURE" (registered trademark) 819 (phenylbis(2,4,6-trimethylbenzoyl)phosphine oxide) (acylphosphine oxide-based polymerization initiator), manufactured by Ciba Specialty Chemicals Inc.
TAA: Non-polymerizable solvent, tert-amyl alcohol, manufactured by Tokyo Chemical Industry Co., Ltd.

### <Method for preparation of phosphate buffered saline solution (PBS)>

In a 2 L Erlenmeyer flask, 16 g of sodium chloride, 0.4 g of potassium chloride, 2.88 g of disodium hydrogen phosphate, 0.48 g of potassium dihydrogen phosphate and 2 L of RO water were weighed. A stirrer was put in the flask and the mixture was well stirred, and after confirming that the reagent is dissolved, filtration was performed using a polyether sulfone filter (EXPRESS PLUS PES 22UM 45MM, manufactured by Merck KGaA) and a vacuum pump to obtain a phosphate buffered saline solution (PBS).

### <Preparation of silicone member A>

2.2 g of MEA, 4.3 g of FM0711, 2.5 g of DMAA, 1.0 g of 164B, 0.05 g of NB, 0.002 g of RB, 0.02 g of IC-819 and 4.0 g of TAA were well mixed. This mixture was filtered through a membrane filter (0.45 um) to remove an insoluble substance to obtain a monomer composition. This monomer composition was injected into molds, and polymerized by light irradiation (1.01 mW/cm², 20 min) using a fluorescent lamp (manufactured by Toshiba Corporation, FL-6D, daylight color, 6 W, 4 fibers) under a nitrogen atmosphere. After polymerization, the molds was immersed in a mixed solution of isopropyl alcohol and water in a ratio of 55 : 45 (volume ratio before mixing) and heated at 60°C for 1 hour to remove a molded body. Thereafter, the molded body thus obtained was immersed in fresh isopropyl alcohol and water in a ratio of 55 : 45 (volume ratio before mixing) and heated at 60°C for 3 hours, followed by extraction.

The molded body obtained after the extraction was immersed in a mixed solution of isopropyl alcohol and water in a ratio of 3 : 7 (volume ratio before mixing) at room temperature for 30 minutes, and then immersed in PBS to be left standing and stored overnight, thereby obtaining a circular sheet-shaped silicone member A having a diameter of 13 mm and a thickness of about 100 um. The surface contact angle A of the silicone member A was 115° as measured by a method to be described later, and high water repellency was exhibited.

### [Example 1]

(A) The silicone member A was immersed in a 0.25 N aqueous sodium hydroxide solution, and heated at 60°C for 30 minutes to perform a hydrolysis reaction. After cooling to room temperature, the silicone member A was taken out from the solution, and lightly swing-washed in PBS. (B) A 1 mass% "Carbodilite" (registered trademark) V-02 aqueous solution (polycarbodiimide, weight average molecular weight 86,000, pH = 8.2, manufactured by Nisshinbo Chemical Inc.) was prepared, and the silicone member A was immersed in the aqueous solution at 60°C for 30 minutes. After cooling, the silicone member A was taken out from the aqueous solution, and lightly swing-washed in PBS. (C) An aqueous solution was prepared by mixing 1 mass% of an aqueous solution of 2-methoxyethyl acrylate/N,N-dimethylacrylamide/acrylic acid copolymer (copolymerization ratio 2/7/1 [molar ratio], weight average molecular weight 1,040,000) and 1 mass% of an aqueous solution of polyacrylic acid (product name AC-10 H, manufactured by Toagosei Company, Limited) at a ratio of 2 : 1.7 (mass ratio). The polyacrylic acid is a polymer having a carboxylic acid group. The silicone member A was immersed in the mixed aqueous solution at 60°C for 30 minutes. After cooling to room temperature, the silicone member A was taken out from the mixed aqueous solution, and lightly swing-washed in PBS. The silicone member was immersed in clean PBS, heat-sterilized at 121°C for 30 minutes, and stored at room temperature to obtain a coated silicone member.

The weight average molecular weight of the used polymer (including polycarbodiimide) was measured under the following conditions.

### (GPC measurement conditions)

Apparatus: Prominence GPC system manufactured by Shimadzu Corporation
Pump: LC-20AD, Autosampler: SIL-20AHT
Column oven: CTO-20A, Detector: RID-10A
Column: GMPXW, manufactured by Tosoh Corporation (7.8 mm in inner diameter × 30 cm, particle diameter of 13 mm) Solvent: water/methanol = 1/1 (0.1 N lithium nitrate is added)
Flow rate: 0.5 mL/minute
Measurement time: 30 minutes, Sample concentration: 0.1 mass%, Injection amount: 20 µL
Standard sample: Polyethylene oxide standard sample, manufactured by Agilent Technologies, Inc. (100 to 1,258,000)

### <(a) Measurement of surface contact angle>

The coated silicone member is immersed in PBS at 25°C for one day or more. After taking the coated silicone member out of the PBS at 25°C, the medical device was immediately sandwiched between two pieces of water absorbent gauze (for example, "Haize (registered trademark)" Gauze VP-150: manufactured by OZU CORPORATION) from above and below, and then water on the surface was removed by lightly pressing. The contact angle was measured as quickly as possible using Automatic Contact Angle Drop Master DM-500 (manufactured by Kyowa Interface Science Co., Ltd.). PBS was used as a droplet for measurement. The surface contact angle was measured 30 seconds after the droplet (1 µL) was brought into contact.

### <(b) Durability test: Surface contact angle>

A solution (artificial tear) was prepared by stirring and dissolving 0.06 g of lysozyme (Albumen derived, manufactured by FUJIFILM Wako Pure Chemical Corporation), 0.08 g of γ-lactoglobulin (Human serum-derived, manufactured by FUJIFILM Wako Pure Chemical Corporation), and 0.015 g of mucin (Bovine submandibular gland derived, manufactured by FUJIFILM Wako Pure Chemical Corporation) in 100 g of PBS. The coated silicone member was immersed in artificial tears in a beaker at room temperature for 24 hours or more. Subsequently, the coated silicone member was pulled up from the artificial tears, placed on the palm, and 5 to 6 drops of artificial tears were dropped onto the coated silicone member with a pipette. The surface of the coated silicone member was scrubbed and washed back and forth 200 times with the palm and the forefinger pad, and immersed in PBS. Thereafter, the surface contact angle was measured.

### <(c) Hydrophilicity evaluation>

0.015 g of Sudan Black B (manufactured by FUJIFILM Wako Pure Chemical Corporation) and 3 g of D-alpha-Tocoferol (manufactured by Tokyo Chemical Industry Co., Ltd.) were weighed in a vial and thoroughly stirred to prepare a Sudan Black solution. The coated silicone member was removed from PBS and immersed in the Sudan Black solution for 5 minutes. The coated silicone member was swung and washed in warm water at 50°C placed in a 100 mL beaker, and then the coated silicone member was taken out and subjected to the following visual evaluation. In the following evaluation criteria, the surface hydrophilicity of A is most maintained and is good, and the surface hydrophilicity of E is lost and is most poor.
A: Dyeing is not observed on the surface of the coated silicone member.
B: Dyeing is observed in a very small amount of punctate or coated silicone member ends.
C: An area of 10% or more and less than 30% of the surface of the coated silicone member is dyed.
D: An area of 30% or more and less than 90% of the surface of the coated silicone member is dyed.
E: An area of 90% or more of the surface of the coated silicone member is dyed.

### <(d) Durability test: Hydrophilicity evaluation>

The coated silicone member is immersed in PBS in advance. The coated silicone member was pulled up from the PBS, placed on the palm, and 5 to 6 drops of the PBS were dropped onto the coated silicone member with a pipette. The surface of the coated silicone member was scrubbed and washed back and forth 200 times with the palm and the forefinger pad, and immersed in PBS. 0.015 g of Sudan Black B (manufactured by FUJIFILM Wako Pure Chemical Corporation) and 3 g of D-alpha-Tocoferol (manufactured by Tokyo Chemical Industry Co., Ltd.) were weighed in a vial and thoroughly stirred to prepare a Sudan Black solution. The coated silicone member was removed from PBS and immersed in the Sudan Black solution for 5 minutes. The coated silicone member was swung and washed in warm water at 50°C placed in a 100 mL beaker, and then the coated silicone member was taken out and subjected to the following visual evaluation. In the following evaluation criteria, the surface hydrophilicity of A is most maintained and is good, and the surface hydrophilicity of E is lost and is most poor.
A: Dyeing is not observed on the surface of the coated silicone member.
B: Dyeing is observed in a very small amount of punctate or coated silicone member ends.
C: An area of 10% or more and less than 30% of the surface of the coated silicone member is dyed.
D: An area of 30% or more and less than 90% of the surface of the coated silicone member is dyed.
E: An area of 90% or more of the surface of the coated silicone member is dyed.

### <Appearance inspection>

The coated silicone member was immersed in PBS in a glass petri dish, and the surface was visually observed by irradiating the coated silicone member with an LED lamp from above. In the following evaluation criteria, A has the best appearance, and E has the worst appearance.
A: The coated silicone member surface is uniform and transparent.
B: The surface of the coated silicone member is uniform, but very little diffuse reflection of light is observed.
C: The surface of the coated silicone member is uniform, but slight diffuse reflection of light is observed.
D: Irregularity is observed on a part of the surface of the coated silicone member, and diffuse reflection of light is observed.
E: Irregularity is observed on the entire of the surface of the coated silicone member, and diffuse reflection of light is observed.

The results of the above measurement and test are shown in Table 1.

### [Example 2]

Except that a 1 mass% "Carbodilite" E-02 (Polycarbodiimide, pH = 8.5, manufactured by Nisshinbo Chemical Inc.) aqueous solution as an emulsion was used in place of the 1 mass% "Carbodilite" V-02 aqueous solution, the same manner as in Example 1 was performed to coat and prepare a silicone member. The pH of the solution was measured at Eutech pH 2700.

### [Example 3]

Except that a 1 mass% "Carbodilite" V-02 (polycarbodiimide, weight average molecular weight 86,000, pH = 8.8, manufactured by Nisshinbo Chemical Inc.) aqueous solution was used in place of the 7 mass% "Carbodilite" V-02 aqueous solution, the same manner as in Example 1 was performed.

### [Example 4]

Except that a 1 mass% "Carbodilite" V-02 (polycarbodiimide, weight average molecular weight 86,000, pH = 8.7, manufactured by Nisshinbo Chemical Inc.) aqueous solution was used in place of the 3 mass% "Carbodilite" V-02 aqueous solution, the same manner as in Example 1 was performed.

### [Example 5]

Except that a 1 mass% "Carbodilite" V-02 (polycarbodiimide, weight average molecular weight 86,000, pH = 7.8, manufactured by Nisshinbo Chemical Inc.) aqueous solution was used in place of the 0.05 mass% "Carbodilite" V-02 aqueous solution, the same manner as in Example 1 was performed.

### [Example 6]

Except that a 1 mass% "Carbodilite" V-02 (polycarbodiimide, weight average molecular weight 86,000, pH = 7.7, manufactured by Nisshinbo Chemical Inc.) aqueous solution was used in place of the 0.03 mass% "Carbodilite" V-02 aqueous solution, the same manner as in Example 1 was performed.

### [Example 7]

Except that a 1 mass% "Carbodilite" V-02 (Weight average molecular weight 86,000, pH = 7.5, manufactured by Nisshinbo Chemical Inc.) aqueous solution was used in place of the 0.005 mass% "Carbodilite" V-02 aqueous solution, the same manner as in Example 1 was performed.

### [Example 8]

This example was performed in the same manner as in Example 1 except that a solution at 40°C was used in each of the steps (A), (B), and (C).

### [Example 9]

This example was performed in the same manner as in Example 1 except that a solution at 70°C was used in each of the steps (A), (B), and (C).

### [Example 10]

This example was performed in the same manner as in Example 1 except that a solution at room temperature was used in each of the steps (A), (B), and (C).

### [Example 11]

This example was performed in the same manner as in Example 5 except that a solution at 50°C was used in each of the steps (A), (B), and (C).

### [Example 12]

This example was performed in the same manner as in Example 1 except that a solution at 90°C was used in each of the steps (A), (B), and (C).

### [Example 13]

This example was performed in the same manner as in Example 1 except that a 1 mass% N,N-dimethylacrylamide/acrylic acid copolymer (copolymerization ratio 2/1 [molar ratio]) aqueous solution was used instead of the mixed aqueous solution of the 2-methoxyethyl acrylate/N,N-dimethylacrylamide/acrylic acid copolymer (copolymerization ratio 2/7/1 [molar ratio], weight average molecular weight 1,040,000) and polyacrylic acid (Product name AC-10 H, manufactured by Toagosei Co., Ltd., weight average molecular weight 380,000).

### [Example 14]

This example was performed in the same manner as in Example 1, except that a solution in which a sodium hydroxide powder was gradually added to a 1 mass% "Carbodilite" V-02 (Polycarbodiimide manufactured by Nisshinbo Chemical Inc., weight average molecular weight: 86,000) aqueous solution to have a pH of 9.5 was used in place of a 1 mass% "Carbodilite" V-02 aqueous solution.

### [Example 15]

This example was performed in the same manner as in Example 1, except that a solution in which a sodium hydroxide powder was gradually added to a 1 mass% "Carbodilite" V-02 (Polycarbodiimide manufactured by Nisshinbo Chemical Inc., weight average molecular weight: 86,000) aqueous solution to have a pH of 10.5 was used in place of a 1 mass% "Carbodilite" V-02 aqueous solution.

### [Example 16]

This example was performed in the same manner as in Example 1, except that a solution was used in which citric acid was gradually added to a 1 mass% "Carbodilite" V-02 aqueous solution to have a pH of 6.5, in place of the 1 mass% "Carbodilite" V-02 aqueous solution.

### [Example 17]

This example was performed in the same manner as in Example 5, except that (B) a 1 mass% "Carbodilite" V-02-L (polycarbodiimide, weight average molecular weight 42,000, manufactured by Nisshinbo Chemical Inc.) aqueous solution was used in place of the 1 mass% "Carbodilite" V-02 aqueous solution.

### [Example 18]

2.2 g of MEA, 4.3 g of FM0711, 2.5 g of DMAA, 1.0 g of 164B, 0.05 g of NB, 0.002 g of RB, 0.02 g of IC-819 and 4.0 g of TAA were well mixed. This mixture was filtered through a membrane filter (0.45 um) to remove an insoluble substance to obtain a monomer composition. This monomer composition was injected into a pair of molds, and polymerized by light irradiation (1.01 mW/cm², 20 min) using a fluorescent lamp (manufactured by Toshiba Corporation, FL-6D, daylight color, 6 W, 4 fibers) under a nitrogen atmosphere. Only one mold was removed. (A) An isopropyl alcohol : water = 55 : 45 (volume ratio before mixing) solution of 0.05 N sodium hydroxide was prepared. In this solution, the silicone member A fixed to the other mold was heated at 60°C for 1 hour to perform hydrolysis reaction of the silicone member A and peeling from the mold. Thereafter, the silicone member A thus obtained was immersed in fresh isopropyl alcohol and water in a ratio of 55 : 45 (volume ratio before mixing) and heated at 60°C for 3 hours, followed by extraction. (B) A 1 mass% "Carbodilite" V-02 aqueous solution was prepared, and the silicone member A was immersed in the aqueous solution at 60°C for 30 minutes. After cooling, the silicone member A was taken out from the aqueous solution, and lightly swing-washed in PBS. (C) An aqueous solution was prepared by mixing 1 mass% of an aqueous solution of 2-methoxyethyl acrylate/N,N-dimethylacrylamide/acrylic acid copolymer (copolymerization ratio 2/7/1 [molar ratio], weight average molecular weight 1,040,000) and 1 mass% of an aqueous solution of polyacrylic acid (product name AC-10 H, manufactured by Toagosei Company, Limited) at a ratio of 2 : 1.7 (mass ratio). The polyacrylic acid is a polymer having a carboxylic acid group. The mixed aqueous solution was heated at 60°C for 30 minutes to immerse the silicone member A therein. After cooling to room temperature, the silicone member A was taken out from the mixed aqueous solution, and lightly swing-washed in PBS. The silicone member was immersed in new PBS, heat-sterilized at 121°C for 30 minutes, and stored at room temperature to obtain a coated silicone member. The results of performing the same measurement and test as in Example 1 are shown in Table 1.

### [Example 19]

This example was performed in the same manner as in Example 18 except that an isopropyl alcohol : water = 55 : 45 (volume ratio before mixing) solution of 0.01 N sodium hydroxide was prepared in step of (A).

### [Example 20]

(A) The silicone member A was immersed in a 0.25 N aqueous sodium hydroxide solution, and heated at 60°C for 30 minutes to perform a hydrolysis reaction. After cooling to room temperature, the silicone member A was taken out from the solution, and lightly swing-washed in PBS.

(D) A mixed aqueous solution was prepared by dissolving 1 mass% of Carbodilite" (registered trademark) V-02 (polycarbodiimide, weight average molecular weight 86,000, pH = 8.2, manufactured by Nisshinbo Chemical Inc.), 1 mass% of a 2-methoxyethyl acrylate/N,N-dimethylacrylamide/acrylic acid copolymer (copolymerization ratio 2/7/1 [molar ratio], weight average molecular weight 1,040,000), and 0.85 mass% of polyacrylic acid (product name AC-10 H, manufactured by Toagosei Company, Limited). The silicone member A was immersed in the mixed aqueous solution at 60°C for 30 minutes. After cooling, the silicone member A was taken out from the mixed aqueous solution, and lightly swing-washed in PBS. The silicone member was immersed in clean PBS, heat-sterilized at 121°C for 30 minutes, and stored at room temperature to obtain a coated silicone member. The results of performing the same measurement and test as in Example 1 are shown in Table 1.

### [Comparative Example 1]

(B) A 1 mass% "Carbodilite" V-02 aqueous solution was prepared, and the silicone member A was immersed in the aqueous solution at 60°C for 30 minutes. After cooling, the silicone member A was taken out from the aqueous solution, and lightly swing-washed in PBS. (C) An aqueous solution was prepared by mixing 1 mass% of an aqueous solution of 2-methoxyethyl acrylate/N,N-dimethylacrylamide/acrylic acid copolymer (copolymerization ratio 2/7/1 [molar ratio], weight average molecular weight 1,040,000) and 1 mass% of an aqueous solution of polyacrylic acid (product name AC-10 H, manufactured by Toagosei Company, Limited) at a ratio of 2 : 0.3 (mass ratio). The polyacrylic acid is a polymer having a carboxylic acid group. The mixed aqueous solution was heated at 60°C for 30 minutes to immerse the silicone member A therein. After cooling to room temperature, the silicone member A was taken out from the solution, and lightly swing-washed in PBS. The silicone member was immersed in new PBS, heat-sterilized at 121°C for 30 minutes, and stored at room temperature to obtain a coated silicone member. The results of performing the same measurement and test as in Example 1 are shown in Table 1.

### [Comparative Example 2]

(A) The silicone member A was immersed in a 0.25 N aqueous sodium hydroxide solution, and heated at 60°C for 30 minutes to perform a hydrolysis reaction. After cooling to room temperature, the silicone member A was taken out from the solution, and lightly swing-washed in PBS. (C) An aqueous solution was prepared by mixing 1 mass% of an aqueous solution of 2-methoxyethyl acrylate/N,N-dimethylacrylamide/acrylic acid copolymer (copolymerization ratio 2/7/1 [molar ratio], weight average molecular weight 1,040,000) and 1 mass% of an aqueous solution of polyacrylic acid (product name AC-10 H, manufactured by Toagosei Company, Limited) at a ratio of 2 : 0.3 (mass ratio). The polyacrylic acid is a polymer having a carboxylic acid group. The mixed aqueous solution was heated at 60°C for 30 minutes to immerse the silicone member A therein. After cooling to room temperature, the silicone member A was taken out from the mixed aqueous solution, and lightly swing-washed in PBS. The silicone member was immersed in new PBS, heat-sterilized at 121°C for 30 minutes, and stored at room temperature to obtain a coated silicone member. The results of performing the same measurement and test as in Example 1 are shown in Table 1.

### [Comparative Example 3]

(A) The silicone member A was immersed in a 0.25 N aqueous sodium hydroxide solution, and heated at 60°C for 30 minutes to perform a hydrolysis reaction. After cooling to room temperature, the silicone member A was taken out from the solution, and lightly swing-washed in PBS. (B) A 1 mass% "Carbodilite" V-02 aqueous solution was prepared, and the silicone member A was immersed in the aqueous solution at 60°C for 30 minutes. After cooling, the silicone member A was taken out from the aqueous solution, and lightly swing-washed in PBS. The silicone member was immersed in new PBS, heat-sterilized at 121°C for 30 minutes, and stored at room temperature to obtain a coated silicone member. The results of performing the same measurement and test as in Example 1 are shown in Table 1.

**[Table 1]**

| | (a) Contact angle [°] | (b) Durability test: Contact angle [°] | (c) Hydrophilicity | (d) Durability test: Hydrophilicity | (e) Appearance |
|---|---|---|---|---|---|
| Example 1 | 45 | 61 | B | C | C |
| Example 2 | 58 | 60 | A | A | C |
| Example 3 | 55 | 72 | A | C | E |
| Example 4 | 55 | 71 | A | B | D |
| Example 5 | 60 | 62 | A | A | A |
| Example 6 | 72 | 75 | A | A | A |
| Example 7 | 80 | 85 | C | C | A |
| Example 8 | 45 | 82 | A | C | C |
| Example 9 | 72 | 75 | B | B | C |
| Example 10 | 68 | 105 | A | D | C |
| Example 11 | 74 | 88 | B | C | A |
| Example 12 | 85 | 82 | B | C | B |
| Example 13 | 62 | 65 | B | B | C |
| Example 14 | 65 | 72 | A | C | B |
| Example 15 | 72 | 79 | A | D | A |
| Example 16 | 48 | 70 | B | C | C |
| Example 17 | 75 | 77 | B | B | A |
| Example 18 | 41 | 65 | B | B | C |
| Example 19 | 45 | 68 | B | A | A |
| Example 20 | 62 | 70 | B | C | C |
| Comparative Example 1 | 112 | 114 | E | E | A |
| Comparative Example 2 | 87 | 95 | C | E | A |
| Comparative Example 3 | 115 | 117 | E | E | A |

## Claims

1. A method for producing a coated silicone member comprising:
(A) a decomposition reaction step of decomposing a surface of a silicone member;
(B) a contact step b of bringing the silicone member whose surface is decomposed into contact with a solution b containing polycarbodiimide; and
(C) a contact step c of bringing the silicone member whose surface is decomposed into contact with a solution c containing a polymer C,
wherein the polymer C has an alkylamide group and a hydroxyl group.

2. The method for producing a coated silicone member according to claim 1, wherein a temperature of the solution b and/or the solution c is 50°C to 80°C.

3. The method for producing a coated silicone member according to claim 1 or 2, wherein the solution b is an emulsion.

4. The method for producing a coated silicone member according to any one of claims 1 to 3, wherein the solution c further contains a polymer having a carboxylic acid group.

5. A method for producing a coated silicone member comprising:
(A) a decomposition reaction step of decomposing a surface of a silicone member; and
(D) a contact step d of bringing the silicone member whose surface is decomposed into contact with a solution d containing polycarbodiimide and a polymer C,
wherein the polymer C has an alkylamide group and a hydroxyl group.

6. The method for producing a coated silicone member according to claim 5, wherein a temperature of the solution d is 50°C to 80°C.

7. The method for producing a coated silicone member according to claim 5 or 6, wherein the solution d is an emulsion.

8. The method for producing a coated silicone member according to any one of claims 5 to 7, wherein the solution d further contains a polymer having a carboxylic acid group.

9. The method for producing a coated silicone member according to any one of claims 1 to 8, wherein the polycarbodiimide has a weight average molecular weight of 45,000 to 200,000.

10. The method for producing a coated silicone member according to any one of claims 1 to 9, wherein the polymer C further has a 2-alkoxyethyl group.

11. The method for producing a coated silicone member according to any one of claims 1 to 10, wherein the decomposition method in the decomposition reaction step (A) is hydrolysis.

12. The method for producing a coated silicone member according to any one of claims 1 to 11, further comprising a fixing step of fixing the silicone member to a surface of a substrate (X).
